(19) Europäisches Patentamt
European Patent Office
Office européen des brevets

(11) Publication number: **0 377 413 B1**

(12) # EUROPEAN PATENT SPECIFICATION

(45) Date of publication of patent specification : **31.03.93 Bulletin 93/13**

(51) Int. Cl.$^5$ : **A61K 31/445,** A61K 9/00, A61K 9/06

(21) Application number : **89830004.1**

(22) Date of filing : **04.01.89**

(54) **Pharmaceutical compositions having topical use comprising Ticlopidine.**

(43) Date of publication of application : **11.07.90 Bulletin 90/28**

(45) Publication of the grant of the patent : **31.03.93 Bulletin 93/13**

(84) Designated Contracting States : **AT BE CH DE FR GB IT LI LU NL SE**

(56) References cited :
**GB-A- 2 038 625**
**GB-A- 2 177 917**
**CHEMICAL ABSTRACTS, vol. 104, no. 23, 9th June 1986, page 191, abstract no. 201177c, Columbus, Ohio, US; A. MORITA et al.: "Beneficial effects of sodium salt of 17(R)-methyl-20-isopropylidenecarbacyclin on experimentally-induced ischemic hind limb lesions and blood viscosity"**

(56) References cited :
**CHEMICAL ABSTRACTS, vol. 104, no. 5, 3rd February 1986, page 37, abstract no. 28599s, Columbus, Ohio, US; G. DAVI et al.: "Inhibition of platelet function by ticlopidine in arteriosclerosis obliterans of the lower limbs"**
**CHEMICAL ABSTRACTS, vol. 102, no. 17, 29th April 1985, page 31, abstract no. 142934f, Columbus, Ohio, US; A. BERNAT et al.: "Antithrombotic effect of ticlopidine in a platelet-independent model of venous thrombosis"**

(73) Proprietor : **ISTITUTO BIOCHIMICO NAZIONALE SAVIO S.r.l.**
**Via E. Bazzano, 14**
**I-16019 Ronco Scrivia (Genova) (IT)**

(72) Inventor : **Stefanelli, Emilio**
**Via Mazzini, 7**
**Pisa (IT)**

(74) Representative : **Bianchetti, Giuseppe**
**Studio Consulenza Brevettuale Via Rossini, 8**
**I-20122 Milan (IT)**

EP 0 377 413 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to pharmaceutical compositions for topical use containing 5-(2-chlorobenzyl)-4,5,6,7-tetrahydro-thieno(3.2-c)-pyridine, of formula I:

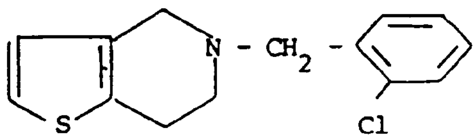

(I)

Compound (I), internationally known as ticlopidine, is used by systemic route as a drug having platelet anti-aggregating anti-thrombotic activity.

Now it has been found that compositions for topical use containing ticlopidine, like ointments, lotions andin particular - creams, have a great therapeutical activity in treating limbs ischemias; vessels alterations (thrombophlebitis and phlebothrombosis, post-phlebitis syndromes, varixes, stasis edemas); hematomas, superficial traumas and swellings.

Ticlopidine therapeutical activity by topical route is surprising because in literature this kind of activity is unknown. Furthermore it's known that many drugs which are active by systemic route are inactive or practically inactive by topical route.

On the other hand topical administration has - where it is possible ot use it - clear advantages, for instance the use of less quantity of active principle and, particularly, the possibility to avoid that notable concentrations of the active principle enter blood circulation.

The compositions according to the invention contain ticlopidine (as hydrochloride) from 1 to 10% and preferably from 2 to 6% in weight, together with excipients and preservatives already known in pharmaceutical technique.

Creams with 3 - 5% of active principle (in form of hydrochloride) as shown hereinbelow, are an example.

### EXAMPLE 1

**3% CREAM**

| 100 g cream composition | |
|---|---|
| TICLOPIDINE | 3 g |
| TEFOSE 63® | 20 g |
| LABRAFIL M 2130 CS® | 3 g |
| WHITE MINERAL OIL | 3 g |
| BUTYLHYDROXYANISOLE | 0.05 g |
| METHYL p-HYDROXYBENZOATE | 0.1 g |
| PROPYL p-HYDROXYBENZOATE | 0.05 g |
| WATER (to 100 g) | 70.80 g |

**EXAMPLE 2**

**5% CREAM**

| | |
|---|---|
| 100 g cream composition | |
| TICLOPIDINE | 5 g |
| TEFOSE 63® | 20 g |
| LABRAFIL M 2130 CS® | 3 g |
| WHITE MINERAL OIL | 3 g |
| BUTYLHYDROXYANISOLE | 0.05 g |
| METHYL p-HYDROXYBENZOATE | 0.1 g |
| PROPYL p-HYDROXYBENZOATE | 0.05 g |
| WATER (to 100 g) | 68.80 g |

**EXAMPLE 3**

**8% CREAM**

| | |
|---|---|
| 100 g composition | |
| TICLOPIDINE | 3 g |
| TEFOSE 63 ® | 19 g |
| LABRAFIL M 2130 CS® | 3 g |
| DERMOIL ® | 3 g |
| METHYL p-OXYBENZOATE | 0.05 g |
| PROPYL p-OXYBENZOATE | 71.85 g |

**EXAMPLE 4**

**5% CREAM**

| | |
|---|---|
| 100 g composition | |
| TICLOPIDINE | 5 g |
| TEFOSE 63® | 19 g |
| LABRAFIL M 2130 CS® | 3 g |
| DERMOIL® | 3 g |
| METHYL p-OXYBENZOATE | 0.1 g |
| PROPYL p-OXYBENZOATE | 0.05 g |
| WATER (to 100 g) | 69.85 g |

**EXAMPLE 5**

**3% CREAM**

| | |
|---|---|
| 100 g cream composition | |
| TICLOPIDINE | 3 g |
| XALIFIN 15® | 20 g |
| GLUCAM E 20® | 3 g |
| CELLOSAN ® | 0.4 g |
| METHYL p-OXYBENZOATE | 0.1 g |
| PROPYL p-OXYBENZOATE | 0.05 g |
| WATER (to 100 g) | 73.45 g |

**EXAMPLE 6**

**5% CREAM**

| | |
|---|---|
| 100 g cream composition | |
| TICLOPIDINE | 3 g |
| XALIFIN 15® | 20 g |
| GLUCAM E 20® | 3 g |
| CELLOSAN ® | 0.4 g |
| METHYL p-OXYBENZOATE | 0.1 g |
| PROPYL p-OXYBENZOATE | 0.05 g |
| WATER (to 100 g) | 73.45 g |

The pharmaceutical compositions of the invention allow a quick and remarkable attenuation of the symptoms, that then completely disappear, in 80-90% of the cases in which they have been experimented. These cases included thrombophlebitis and phlebothrombosis, acentrated limbs ischemias; and also hematomas caused by contusions and vessel fragility, swellings of various kinds and varixes.

Tolerability controls, carried out using the 5% active principle creams, (see Example 4) preceeded the above mentioned experiments.

The following tables summarize the results of the "acute study" (primary irritation and light-sensitive capacity test in guinea-pigs)

TABLE 1 - Irritation test on non-treated skin of guinea-pigs treated with 2 g/kg and 6 g/kg of 5% Ticlopidine cream.

| Animal n. | Treatment | Results after 24 hours | | Results after 48 hours | |
|---|---|---|---|---|---|
| | | Erythema | Edema | Erythema | Edema |
| 1 | Cream excipients | 0 | 0 | 0 | 0 |
| 2 | Cream excipients | 1 | 0 | 0 | 0 |
| 3 | Cream excipients | 0 | 0 | 0 | 0 |
| 4 | Cream excipients | 0 | 0 | 0 | 0 |
| 5 | Cream excipients | 0 | 0 | 0 | 0 |
| 6 | Cream excipients | 0 | 0 | 0 | 0 |
| 7 | Ticlopidine 2 g/kg | 0 | 0 | 0 | 0 |
| 8 | Ticlopidine 2 g/kg | 0 | 0 | 0 | 0 |
| 9 | Ticlopidine 2 g/kg | 0 | 0 | 0 | 0 |
| 10 | Ticlopidine 2 g/kg | 0 | 0 | 0 | 0 |
| 11 | Ticlopidine 2 g/kg | 1 | 0 | 0 | 0 |
| 12 | Ticlopidine 2 g/kg | 0 | 0 | 0 | 0 |
| 13 | Ticlopidine 6 g/kg | 0 | 0 | 0 | 0 |
| 14 | Ticlopidine 6 g/kg | 0 | 0 | 0 | 0 |
| 15 | Ticlopidine 6 g/kg | 1 | 0 | 0 | 0 |
| 16 | Ticlopidine 6 g/kg | 0 | 0 | 0 | 0 |
| 17 | Ticlopidine 6 g/kg | 0 | 0 | 0 | 0 |
| 18 | Ticlopidine 6 g/kg | 1 | 0 | 0 | 0 |

**Note:** For the erythema the following classification has been adopted: 0 = absent; 1 = slight; 2 = well defined; 3 = from moderate to serious; 4 = serious, with deep lesions. Edema: 0 = absent; 1 = slight; 2

= slight with raised margins; 3 = moderate with raised area for nearly 1 $mm^2$.

TABLE 2 - Primary irritation test on scarified skin of guinea - pigs treated with 2 g/kg and 6 g/kg of 5% Ticlopidine cream.

| Animal n. | Treatment | Results after 24 hours | | Results after 48 hours | |
|---|---|---|---|---|---|
| | | Erythema | Edema | Erythema | Edema |
| 1 | Cream excipients | 0 | 0 | 0 | 0 |
| 2 | Cream excipients | 1 | 0 | 0 | 0 |
| 3 | Cream excipients | 0 | 0 | 0 | 0 |
| 4 | Cream excipients | 1 | 0 | 0 | 0 |
| 5 | Cream excipients | 0 | 0 | 0 | 0 |
| 6 | Cream excipients | 1 | 0 | 1 | 0 |
| 7 | Ticlopidine 2 g/kg | 0 | 0 | 0 | 0 |
| 8 | Ticlopidine 2 g/kg | 0 | 0 | 0 | 0 |
| 9 | Ticlopidine 2 g/kg | 1 | 0 | 1 | 0 |
| 10 | Ticlopidine 2 g/kg | 0 | 0 | 0 | 0 |
| 11 | Ticlopidine 2 g/kg | 0 | 0 | 0 | 0 |
| 12 | Ticlopidine 2 g/kg | 1 | 0 | 0 | 0 |
| 13 | Ticlopidine 6 g/kg | 0 | 0 | 0 | 0 |
| 14 | Ticlopidine 6 g/kg | 0 | 0 | 0 | 0 |
| 15 | Ticlopidine 6 g/kg | 1 | 0 | 1 | 0 |
| 16 | Ticlopidine 6 g/kg | 0 | 0 | 0 | 0 |
| 17 | Ticlopidine 6 g/kg | 0 | 0 | 0 | 0 |
| 18 | Ticlopidine 6 g/kg | 1 | 0 | 0 | 0 |

**Note:** the classification is the same of Table 1.

TABLE 3 - Photo Patch Test in guinea-pigs treated with 5% Ticlopidine cream.

| Animal n. | Treatment | UVA | | | UVB | | |
|---|---|---|---|---|---|---|---|
| | | 48 h | 72 h | 96 h | 48 h | 72 h | 96 h |
| 1 | Cream excipients | - | - | - | - | - | - |
| 2 | Cream excipients | - | - | - | - | - | - |
| 3 | Cream excipients | - | - | - | - | - | - |
| 4 | Cream excipients | - | - | - | - | - | - |
| 5 | Cream excipients | - | - | - | - | - | - |
| 6 | Cream excipients | - | - | - | - | - | - |
| 7 | Ticlopidine cream | - | - | - | - | - | - |
| 8 | Ticlopidine cream | - | - | - | - | - | - |
| 9 | Ticlopidine cream | - | - | - | - | - | - |
| 10 | Ticlopidine cream | - | - | - | - | - | - |
| 11 | Ticlopidine cream | - | - | - | - | - | - |
| 12 | Ticlopidine cream | - | - | - | - | - | - |

**Note:** A xenon lamp emitting light similar to sun-light of a constant intensity, was used. After determing the minimum erythema dose (DEM) of irradiation, the photostimulation with luminous radiation as such (UVA) or filtered by radiations with < 3200 A (UVB) was carried out. In the first case of 0.75 DEM, in the second for 30 minutes.

As it is stated by Table 1 and 2, the cream turns out to be included among the classifications where it is not considered irritant, while Table 3 shows the complete absence of light-sensitive phenomena.

Furthermore, the local chronic tolerability in rabbits was valued.

The above mentioned cream was applied daily and for one month in a shaven area ( 50 $cm^2$) of whitish rabbits right side. Weekly shavings occurred, while the excipients were tested in the same way on the left side. Tolerability was very good for all the twelve animals; as regards the controls, no variations in weight increase, in hematological and hematochemical pattern, at the macroscopic, weighting and histological exam of the organs, and also of the hemodynamic and breathing parameters were shown.

See, for instance, Table 4-5-6.

**TABLE 4:** Local tolerability of 5% Ticlopidine cream applied on the skin of whitish rabbits for a period of one month.
Weight variations (M+ ES in g)

| Days of treatment | TREATMENT | |
|---|---|---|
| | Control | 5% Ticlopidine cream |
| 0 | 3408 ± 87 | 3433 ± 99 |
| 7 | 3492 ± 81 | 3500 ± 104 |
| 14 | 3585 ± 82 | 3576 ± 109 |
| 21 | 3630 ± 87 | 3593 + 104 |
| 30 | 3700 ± 104 | 3695 ± 111 |

TABLE 5: Local tolerability of 5% Ticlopidine cream applied on the skin of whitish rabbits for a period of one month.
Organs' weight: % values (M + ES)

| Treatment | Liver % | Spleen % | Kidneys % | Heart % | Suprarenal % |
|---|---|---|---|---|---|
| Control | 3,22±0,03 | 0,04±0,33 | 0,42±0,01 | 0,24±0,012 | 12,40±0,91 |
| 5 % Ticlopidine cream | 3,36±0,32 | 0,05±0,024 | 0,39±0,01 | 0,22±0,011 | 12,90±0,60 |

**TABLE 6:** Results of the treatment with 5% Ticlopidine cream on blood pressure, heart rate and breath in anaesthetized rabbits.
Values are expressed as increase or decrease in % as regards the basic values.
3 animals for every group (M + ES)

| Time | Treatment | Blood pressure | Heart rate | Breath | |
|---|---|---|---|---|---|
| | | | | Wideness | Frequency |
| After 15 days | Ticlopidine cream | - 2,5 | + 1,5 | + 2,2 | + 0,3 |
| | control | + 1,3 | - 1,2 | + 1,6 | + 0,2 |
| End of treatment | Ticlopidine cream | + 3,2 | - 0,9 | + 1,6 | - 0,9 |
| | control | - 0,6 | + 1,4 | - 0,5 | + 1 |

## Claims

1. Pharmaceutical compositions adapted only for topical use, containing as active principle 5-(2-chlorobenzyl)-4,5,6,7-tetrahydro-thieno (3,2-c)pyridine (ticlopidine) of formula I:

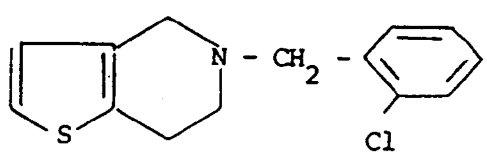

(I)

2. Pharmaceutical compositions according to claim 1, for the treatment of limbs ischemias; vessels alteration (thrombophlebitis, phlebothrombosis, post-phlebitis syndromes, varixes, stasis edema); hematomas, superficial traumas and swellings.

3. Pharmaceutical compositions, according to claims 1 and 2, containing from 1 to 10% in weight of ticlopidine.

4. Pharmaceutical compositions, according to claim 3, containing from 2 to 6% in weight of ticlopidine.

5. Pharmaceutical compositions, according to claim 4, in form of creams containing 3-5% in weight of ticlopidine.

## Patentansprüche

1. Pharmazeutisches Präparat nur für die topische Anwendung, dadurch **gekennzeichnet,** daß es als Wirkstoff 5-(2-Chlorbenzyl)-4,5,6,7-tetrahydrothieno-(3,2-c)-pyridin (Ticlopidin) der Formel I

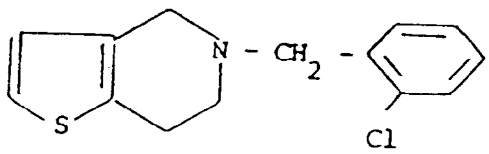

(I)

enthält.

2. Pharmazeutisches Präparat nach Anspruch 1 für die Behandlung von Gliederischemie, Gefäßänderungen (Thrombophlebitis, Phlebothrombose, Post-Phlebitis-Syndrom, Varicen, Staseödem), Hämatomen, oberflächlichen Traumen und Schwellungen.

3. Pharmazeutisches Präparat nach Anspruch 1 und 2, dadurch **gekennzeichnet,** daß es 1 bis 10 Gew.-% Ticlopidin enthält.

4. Pharmazeutisches Präparat nach Anspruch 3, dadurch **gekennzeichnet,** daß es 2 bis 6 Gew.-% Ticlopidin enthält.

5. Pharmazeutisches Präparat nach Anspruch 4, dadurch **gekennzeichnet,** daß es in Form von Cremes vorliegt, die 3 bis 5 Gew.-% Ticlopidin enthalten.

## Revendications

1. Compositions pharmaceutiques destinées exclusivement à l'usage topique, contenant comme principe actif la 5-(2-chlorobenzyl)-4,5,6,7-tétrahydrothiéno[3,2-c]pyridine (ticlopidine) de formule I :

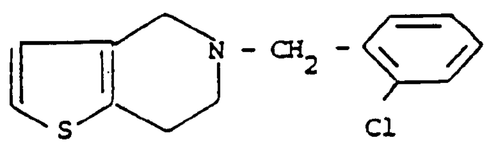

(I)

2. Compositions pharmaceutiques selon la revendication 1, pour le traitement d'ischémies des membres ; d'altération des vaisseaux (thrombophlébite, phlébothrombose, syndromes de post-phlébite, varices, oedème de stase) ; hématomes, traumatismes superficiels et enflures.

3. Compositions pharmaceutiques selon les revendications 1 et 2, contenant 1 à 10 % en poids de ticlopidine.

4. Compositions pharmaceutiques selon la revendication 3, contenant 2 à 6 % en poids de ticlopidine.

5. Compositions pharmaceutiques selon la revendication 4, sous forme de crèmes contenant 3 à 5 % en poids de ticlopidine.